(19) Europäisches Patentamt
European Patent Office
Office européen des brevets

(11) EP 1 411 354 A1

(12) EUROPEAN PATENT APPLICATION
published in accordance with Art. 158(3) EPC

(43) Date of publication:
**21.04.2004 Bulletin 2004/17**

(21) Application number: **02733440.8**

(22) Date of filing: **10.06.2002**

(51) Int Cl.7: **G01N 33/15**, G01N 33/50, A61P 17/00, A61K 49/00, A61K 45/00

(86) International application number:
**PCT/JP2002/005736**

(87) International publication number:
**WO 2002/103351 (27.12.2002 Gazette 2002/52)**

(84) Designated Contracting States:
**AT BE CH CY DE DK ES FI FR GB GR IE IT LI LU MC NL PT SE TR**

(30) Priority: **14.06.2001 JP 2001180366**

(71) Applicant: **SHISEIDO COMPANY, LTD.**
**Chuo-ku, Tokyo 104-8010 (JP)**

(72) Inventors:
- **INOUE, Kaori, c/o Shiseido Research Center Yokohama-shi, Kanagawa 236-8643 (JP)**
- **FUJIWARA, Shigeyoshi, c/o Shiseido Research Center Yokohama-shi, Kanagawa 236-8643 (JP)**
- **DENDA, Mitsuhiro, c/o Shiseido Research Center Yokohama-shi, Kanagawa 236-8643 (JP)**
- **DENDA, Sumiko, c/o Shiseido Research Center Yokohama-shi, Kanagawa 224-8558 (JP)**

(74) Representative: **Henkel, Feiler & Hänzel**
**Möhlstrasse 37**
**81675 München (DE)**

# (54) METHOD OF DETECTING SKIN STIMULATION EFFECT

(57) As a result of studies on a noxious stimulation receptor, it is found out that this stimulation receptor exists in epidermal cells, etc. and changes are observed in intracellular calcium ion concentration depending on the state of this stimulation receptor. The present invention provides a method of detecting a skin stimulation effect based on the relation between the skin stimulation effect of a test substance which is in contact with epithelial cells such as epidermal cells and an increase/decrease in intracellular calcium ion concentration in the epithelial cells.

EP 1 411 354 A1

SEQUENCE LISTING

<110> SHISEIDO COMPANY, LTD.

<120> Method for Detecting Skin Stimulating Action

<130> PSHI400/PCT

<140> EP 02 733 440.8
<141>

<150> PCT/JP02/05736
<151>

<150> JP P2001-180366
<151> 2001-06-14

<160> 7

<170> PatentIn Ver. 2.1

<210> 1
<211> 15
<212> PRT
<213> Rattus sp.

<400> 1
Val Glu Lys Gln Ser Thr Asp Ser Gly Ala Tyr Ser Ile Gly His
  1               5                   10                  15

<210> 2
<211> 22
<212> PRT
<213> Rattus sp.

<400> 2

Tyr Thr Gly Ser Leu Lys Pro Glu Asp Ala Glu Val Phe Lys Asp Ser
1 5 10 15

Met Val Pro Gly Glu Lys
20

<210> 3

<211> 18

<212> PRT

<213> Rattus sp.

<400> 3

Arg Ala Ser Leu Asp Ser Glu Glu Ser Glu Ser Pro Pro Gln Glu Asn
1 5 10 15

Ser Cys

<210> 4

<211> 18

<212> DNA

<213> Rattus sp.

<400> 4

gacatgccac ccagcagg 18

<210> 5

<211> 20

<212> DNA

<213> Rattus sp.

<400> 5

tcaattccca cacacctccc 20

210> 6

<211> 24

<212> DNA

<213> Mus sp.

<400> 6

tggagttctg ggcattaaga tcgg 24

<210> 7

<211> 23

<212> DNA

<213> Mus sp.

<400> 7

ttagtgacca atagaatagg ccc 23

## Description

Technical Field

The present invention relates to a method of detecting specific characteristics of a test substance, and more particularly to a method of detecting the effect of a test substance in stimulating or irritating the skin.

Background Art

As has been known, human pain sensation is induced through activation of a certain sensory nerve terminal by means of various types of stimulation. Pain sensation is essential for human survival, and control of pain is very important in the field of medicine.

Therefore, appropriate control of pain by analgesic means is very important. In order to attain such appropriate control, a mechanism by which human pain sensation is induced must be elucidated, and various analgesic means such as analgesic agents must be developed on the basis of the results of studies performed on the thus-elucidated mechanism.

Conceivably, the human nervous system senses pain when a sensory nerve having a noxious stimulation receptor (a polymodal nociceptor) is activated by noxious stimulation (e.g., stimulation by means of a chemical substance, mechanical stimulation, or thermal stimulation), and the sensory nerve transmits stimulation signals to the spinal cord or the brain.

Recently, the gene for the receptor of capsaicin, which is a primary component of chili pepper and is a fat-soluble, pain stimulation substance, has been isolated, and the capsaicin receptor has been elucidated to be an ion channel having six transmembrane regions and to be activated by means of, in addition to capsaicin, heat or proton, which induces pain.

As has been known, such a noxious stimulation receptor (e.g., a VR1 receptor (vanilloid receptor subtype 1) or a P2X receptor (inotropic purinoreceptor)) is present in specific peripheral nerve cells (cells of unmyelinated C fibers).

Objects of the present invention are to obtain new findings on the basis of the results of studies on the mechanism of human pain sensation, and to provide means which contributes to development of industry through use of the findings.

Disclosure of the Invention

The present inventors have performed studies on the aforementioned noxious stimulation receptors (hereinafter may be referred to as "stimulation receptors"), and as a result have found that the stimulation receptors, which have conventionally been considered to be present merely in specific nerve cells, are also present in epithelial cells such as epidermal cells.

The present inventors have also found that, as in the case of nerve cells, the concentration of intracellular calcium ions in epithelial cells changes through such a stimulation receptor.

In connection with the above finding, the present inventors have found that skin stimulation can be objectively determined by use, as an indication, of an increase/decrease in the concentration of intracellular calcium ions in epithelial cells such as epidermal cells, and that the skin stimulation effect of a test substance can be detected by means of this determination. The present invention has been accomplished on the basis of this finding.

Accordingly, the present invention provides a skin stimulation effect detection method comprising correlating the skin stimulation effect of a test substance which has been brought into contact with epithelial cells such as epidermal keratinocytes with an increase/decrease in the concentration of intracellular calcium ions in the epithelial cells, to thereby detect the skin stimulation effect of the test substance (hereinafter the method may be referred to as "the present detection method").

An increase in the intracellular calcium ion concentration is induced by the mediation of at least two noxious stimulation receptors (i.e., a VR1 receptor and a P2X receptor) in epithelial cells. Therefore, the present detection method is also described as a method of detecting the skin stimulation effect of a test substance by bringing the test substance into contact with epithelial cells such as epidermal keratinocytes, and by correlating an increase/decrease in the concentration of intracellular calcium ions in the epithelial cells with the skin stimulation effect of the test substance; i.e., the effect of the test substance on changing the concentration of intracellular calcium ions in the epithelial cells by the mediation of the VR1 receptor and/or P2X receptor on the epidermis.

Through use of the present detection method, screening of test drugs in relation to skin stimulation (e.g., candidate substances for an analgesic agent or an anti-inflammatory agent) can be carried out in an appropriate and convenient manner.

Accordingly, the present invention also provides a method of screening drugs that are potentially useful for preventing skin stimulation and/or drugs that are potentially useful for preventing epidermal disorder, which comprises using the present detection method as screening means and, as an indication, suppression of an increase in the concentration of intracellular calcium ions in epithelial cells, which increase is caused by a skin stimulation substance (hereinafter the method will may be referred to as "the present screening method").

As used herein, the term "skin stimulation" refers to, for example, itching of the skin, pain of the skin, or irritated skin, which is induced by a chemical substance contained in an external use composition, etc., or by other environmental factors; and the term "epidermal disorder" refers to, for example, inflammation, epidermal proliferative disorder, or cornification disorder.

The aforementioned findings reveal that an increase/decrease in the concentration of intracellular calcium ions in epidermal cells reflects stimulated state of the skin or epidermal disorder in the skin, and thus can be employed as an indication for evaluating conditions of the skin.

Accordingly, the present invention also provides a method of evaluating conditions of the skin, which comprises correlating an increase/decrease in the concentration of intracellular calcium ions in epidermal cells with conditions of the skin (hereafter the method may be referred to as "the present evaluation method").

Brief Description of the Drawings

Fig. 1A shows the results of detection of an increase in the concentration of intracellular calcium ions in epidermal keratinocytes, which increase is caused by capsaicin; and Fig. 1B shows the results of detection of the effect of capsazepine (antagonist) in inhibiting an increase in the intracellular calcium ion concentration.

Fig. 2 shows the results of detection of an increase in the concentration of intracellular calcium ions in epidermal keratinocytes, which increase is caused by ATP or αβ-me ATP, and the results of detection of the effect of TNP-ATP (antagonist) in inhibiting an increase in the intracellular calcium ion concentration.

Fig. 3 shows the results of detection of change in the concentration of intracellular calcium ions in epidermal keratinocytes, which is induced by bradykinin.

Fig. 4 shows the results of detection of change in the concentration of intracellular calcium ions in epidermal keratinocytes, which is induced by lactic acid.

Best Mode for Carrying Out the Invention

Embodiments of the present invention will next be described.

Examples of epithelial cells employed in the present detection method, which constitute an epidermal portion of the skin, include epidermal cells (e.g., epidermal keratinocytes), sebaceous cells, mammary gland cells, small intestine epithelial cells, and mucosal epithelial cells. Of these, epidermal keratinocytes, which are a type of epidermal cells, are preferred epithelial cells employed in the present detection method, since cultured epidermal keratinocytes are readily available, and the present detection method relates to the state of the epidermis. In most cases, the ultimate target of the present detection method is considered to be a human subject. Therefore, preferably, epithelial cells employed in the method are obtained from a human. In the present detection method, generally, cultured epithelial cells are employed. Such cultured epithelial cells can be prepared by means of a customary technique.

For example, in the case where human epidermal keratinocytes are employed as epithelial cells, the keratinocytes are prepared through the following procedure: adipose tissues and blood are removed from excess skin pieces obtained through, for example, plastic surgery, dermatologic procedure, or surgical operation; a portion of the dermis is separated from the resultant skin pieces by means of protease treatment or a similar technique, to thereby prepare a human epidermis sample; epidermal keratinocytes are separated from the epidermis sample, and then subjected to primary culture by means of a customary technique employing, for example, a KGM medium; and the resultant primary culture cells of the human epidermal keratinocytes are subjected to subculture by use of, for example, protease, to thereby prepare cultured human epidermal keratinocytes of interest. Such epidermal keratinocytes are commercially available, and such a commercial product may be employed in the present invention.

Preferably, the present detection method employs the above-prepared cultured epidermal keratinocytes.

In the present detection method, preferably, the concentration of intracellular calcium ions in epithelial cells such as epidermal keratinocytes is determined before and after a test substance is brought into contact with the epithelial cells. Therefore, in general, after the epithelial cells are subjected to means for determining an increase/decrease in the intracellular calcium ion concentration in the cells, the test substance is brought into contact with the epithelial cells.

No particular limitations are imposed on the means for determining an increase/decrease in the intracellular calcium ion concentration, and a typical example of the means is the following treatment: epithelial cells are treated with a reagent which has characteristic of binding to calcium ion (hereinafter, the reagent may be referred to as a "calcium indicator"), to thereby introduce the reagent into the cells. No particular limitations are imposed on the calcium indicator, so long as the concentration of intracellular calcium ions in the cells can be measured by use of the indicator. Examples of the calcium indicator which may be employed include calcium-sensitive fluorescent dyes such as Quin 2, Quin 2-AM, Fura-2, Fura-2-AM, Indo-1, Fluo-3, Fluo-3-AM, and Rhod-2; calcium-sensitive fluorescent proteins such as aequorin; and reagents employed for $^{19}F$ nuclear magnetic resonance spectroscopy, such as 5-FBAPTA. The present detection method may employ epithelial cells into which a calcium-sensitive fluorescent protein such as aequorin has been introduced in advance.

In the present detection method, epithelial cells are subjected to a treatment in accordance with the type of a chosen calcium indicator, the concentration of intracellular calcium ions in the epithelial cells is measured before and after a test substance is brought into contact with the epithelial cells, and an increase/decrease in the intracellular calcium ion concentration, which occurs through contact of the test substance with the epithelial cells, is determined on the basis of the measurement results.

Stimulation receptors which have been found to be present in the surfaces of epithelial cells; i.e., a VR1 receptor (vanilloid receptor subtype 1) and a P2X receptor (inotropic purinoreceptor), can promote influx of calcium ions or hydrogen ions into the cells when a chemical substance which induces noxious stimulation, such as capsaicin or adenosine 5'-triphosphate, acts as an agonist. Through influx of such a chemical substance into the epithelial cells, the concentration of intracellular calcium ions in the cells increases. An increase in the concentration of intracellular calcium ions in the epithelial cells induces pain sensation in the skin, etc., and triggers cornification or proliferation of the skin, etc., as well as secretion of an inflammatory substance. Therefore, an increase/decrease in the intracellular calcium ion concentration can be used as an indication for determining whether or not a phenomenon which directly leads to skin stimulation or epidermal disorder occurs in the epithelial cells.

Thus, in the present detection method, the skin stimulation effect of a test substance which has been brought into contact with epithelial cells such as epidermal keratinocytes is correlated with an increase/decrease in the concentration of intracellular calcium ions in the epithelial cells, to thereby detect the skin stimulation effect of the test substance.

As described above, through use of the present detection method, screening of test drugs in relation to skin stimulation (e.g., candidate substances for an analgesic agent or an anti-inflammatory agent) can be carried out in an appropriate and convenient manner (the present screening method).

Specifically, a test substance which has been elucidated, by means of the present detection method, to have ability to suppress an increase in the concentration of intracellular calcium ions in epithelial cells can be determined, through screening, as a substance exhibiting activity to suppress pain sensation or skin disorder such as inflammation.

In a specific embodiment of the present screening method, a chemical substance which is known to induce noxious stimulation is used as a positive control, and a test sample containing the chemical substance and a test substance to be screened are brought into contact with epithelial cells, and the concentration of intracellular calcium ions in the epithelial cells is measured by means of the present detection method. When the results show that the test sample suppresses an increase in the concentration of intracellular calcium ions in the epithelial cells—which increase occurs in the case of the positive control—the test substance to be screened is determined to exhibit the effect of suppressing skin stimulation or epidermal disorder.

When the effect of decreasing the concentration of intracellular calcium ions in epithelial cells is determined by means of the present detection method without employment of the aforementioned positive control, a test substance exhibiting the effect of suppressing noxious stimulation can be determined through screening by use, as an indication, of a decrease in the intracellular calcium ion concentration.

Thus, there is provided the present screening method; i.e., a method of screening drugs that are potentially useful for preventing skin stimulation and/or drugs that are potentially useful for preventing epidermal disorder, in which the present detection method is employed as screening means, and suppression of an increase in the concentration of intracellular calcium ions in epithelial cells, which increase is caused by a skin stimulation substance, is employed as an indication.

As described above, an increase/decrease in the concentration of intracellular calcium ions in epidermal cells reflects the stimulated state of the skin or epidermal disorder in the skin, and thus can be employed as an indication for evaluating conditions of the skin. In relation to this, the present evaluation method is provided.

In the present evaluation method, in the case of the actual skin, an increase/decrease in the concentration of intracellular calcium ions in epidermal cells can be evaluated through, for example, measurement of the electrical potential of the skin surface. The surface of normal skin contains calcium ions in a high concentration, and thus the skin surface exhibits a negative electrical potential. However, when the skin sustains any damage or when the skin suffers metabolic disorder due to disease, etc., the concentration of calcium ions in the skin surface becomes nearly equal to that of intracellular calcium ions in epidermal cells, and the electrical potential of the skin surface tends to approach zero. Through measurement of such skin surface electrical potential, an increase/decrease in the concentration of intracellular calcium ions in epidermal cells can be evaluated in the actual skin of a subject, whereby conditions of the skin of the subject can be evaluated.

Specifically, in the case where the concentration of intracellular calcium ions in epidermal cells of a subject is higher than a normal level, any inflammation, roughening, or cornification disorder occurs in the skin of the subject, and thus the condition of the skin is evaluated to be undesirable. When the concentration of intracellular calcium ions in epidermal cells of a subject is measured before and after treatment of the skin of the subject, and the intracellular calcium ion concentration is determined to return to a normal level after the skin treatment, efficacy of the skin treatment can be confirmed by means of the present evaluation method. When the present evaluation method is periodically applied to a single subject, the skin conditions of the subject can be investigated with passage of time through evaluation of an increase/decrease in the concentration of intracellular calcium ions in epidermal cells.

## Examples

The present invention will next be described in more detail by way of Examples.

### [Referential Example] Confirmation of existence of stimulation receptor in epidermal cells

Immunostaining

The back skin was collected from a hairless mouse (age: 7 to 10 weeks, HR-1) by means of a customary method, and the back skin was quickly frozen in a metallic jar filled with isopentane which was placed in liquid nitrogen, and stored at - 80°C. The freeze-stored epidermis of the mouse was sliced into pieces having a thickness of 5 μm, and the resultant epidermis slice was fixed with methanol of -20°C for 10 minutes, and then immersed in PBS. Subsequently, the epidermis piece was subjected to blocking by use of a blocking solution [product of Neuromics (USA)] at room temperature for one hour. Thereafter, the resultant epidermis piece was immersed in a diluted solution of an antiserum against a VR1 receptor or a P2X receptor (both of the receptors are found in nerve cells) at 4°C overnight, the antiserum being (a) polyclonal antiserum (product of Neuromics) which had been obtained by immunizing a guinea pig or a rabbit with a peptide which is generally found in a rat P2X3 receptor (one of P2X receptor subtypes), the peptide having the following amino acid sequence: VEKQSTDSGAYSIGH (SEQ ID NO: 1, the amino acid sequence is represented by one-letter code, the same shall apply hereinafter (exclusive of the amino acid sequences shown in the appended sequence listing)), or (b) polyclonal antiserum (product of Neuromics) which had been obtained by immunizing a guinea pig or a rabbit with a peptide of a rat VR1 receptor protein, the peptide having the following C-terminal amino acid sequence: YTGSLKPEDAEVFKDSMVPGEK (SEQ ID NO: 2) and the following N-terminal amino acid sequence: RASLDSEESESPPQENSC (SEQ ID NO: 3). Subsequently, the resultant epidermis piece was washed with PBS containing 0.05% Tween 20 three times for 15 minutes, and then the epidermis piece was subjected to treatment in a solution of a fluorescent secondary antibody (IgG-bound anti-guinea pig or anti-rabbit antibody, product of Molecular Probes, Inc.) at room temperature for one hour. Subsequently, the resultant epidermis piece was washed with PBS containing 0.05% Tween 20 three times for 15 minutes, and then a sample of the epidermis piece was prepared by use of Vectashied (Vector Laboratories, Inc., USA). The epidermis piece sample was subjected to observation within six hours after preparation thereof.

The results reveal that the VR1 receptor and P2X receptor, which are noxious stimulation receptors, are found in the epidermis of the hairless mouse.

RT-PCR assay

The epidermis was separated from the aforementioned skin tissue through incubation carried out in a 10 mM EDTA PBS solution at 37°C for 30 minutes, and the total RNA of the epidermis was isolated as directed in the instructions of ISOGEN (product of ISOGEN). The resultant product was precipitated in ammonium acetate (10 mM) and ethanol. The thus-obtained pellets were suspended in distilled water (10 μl), and a portion (2 μl) of the resultant suspension was subjected to analysis by means of an RT-PCR assay.

For analysis of the VR1 receptor, 5'-GACATGCCACCCAGCAGG (primer 1, SEQ ID NO: 4) and 5'-TCAATTCCCACACACCTCCC (primer 2, SEQ ID NO: 5) were employed in accordance with the nucleotide sequence of the gene encoding a known rat VR1 receptor.

For analysis of the P2X3 receptor, 5'-TGGAGTTCTGGGCATTAAGATCGG (primer 3, SEQ ID NO: 6) and 5'-TTAGTGACCAATAGAATAGGCCC (primer 4, SEQ ID NO: 7) were employed in accordance with the nucleotide sequence of the gene encoding a known mouse P2X3 receptor.

As a result, the positive bands corresponding to the VR1 receptor and P2X3 receptor, which are noxious stimulation receptors, were observed.

The results reveal that the VR1 receptor and P2X receptor are present in epidermal cells as well as in nerve cells, and pain sensation, etc. are induced by the mediation of these noxious stimulation receptors.

[Example] Evaluation of the present detection method and the present screening method

(1) Fundamental test method

(a) Commercially available epidermal keratinocytes (product of Kurabo Industries Ltd.) were subjected to culture by means of a customary method by use of a KGM medium which had been prepared through the following procedure: MCDB153IK (product of Kyokuto Pharmaceutical Industrial Co., Ltd.) (10.93 g), N-2-hydroxyethylpiperazine-N'-2-ethanesulphonic acid (HEPES) (6.7 g), $NaHCO_3$ (1.2 g), insulin (5 mg), hydrocortisone (0.5 mg), transferrin (10 mg), phosphorylethanolamine (14.1 mg), EGF (10 μg), penicillin (100 mg), and kanamycin (60 mg) were dissolved in purified water (1 L); the pH of the resultant solution was adjusted to 7.4 by use of NaOH; the resultant solution was subjected to filter sterilization; and bovine pituitary gland extract (product of Kyokuto Pharmaceutical Industrial Co., Ltd.) (55 mg) was added to the resultant solution immediately before use of the solution. On the day before measurement of calcium ions was carried out, the thus-cultured epidermal keratinocytes were inoculated into a 96-well plate ($2 \times 10^5$ cells/well). On the following day, the aforementioned culture solution was removed from the well plate by use of a pipette such that the cells were not exfoliated from the wells. Subsequently, a balanced salt solution (BSS: an aqueous solution prepared by dissolving NaCl (150 mM), KCl (5 mM), $CaCl_2$ (1.8 mM), $NaH_2PO_4$ (1.2 mM), $MgCl_2$ (1.2 mM), D-glucose (10 mM), and HEPES (25 mM) in water, and adjusting the pH of the resultant solution to 7.4 by use of NaOH) and Fluo3-AM (product of Dainippon Pharmaceutical Co., Ltd.), which is a calcium-sensitive fluorescent dye, were added to the cultured epidermal keratinocytes (total amount of BSS and Fluo3-AM per well: 5 μM), and incubation was carried out at 37°C for 60 minutes, to thereby introduce the fluorescent dye into the cultured epidermal keratinocytes. After completion of introduction of the fluorescent dye, the resultant cells were washed with a PBS(-) solution, and then a fresh BSS was added to the cells, followed by allowing the resultant cells to stand for 15 minutes. Thereafter, the BSS was removed from the cultured epidermal keratinocytes. When the cells were not stimulated, a fresh BSS was added to the cells, whereas when the cells were stimulated by a test substance, the test substance was dissolved in a BSS, and the resultant solution was added to the cells.

The fluorescence intensity was measured over time by means of a customary method by use of a fluorescence microplate reader (485 nm, 538 nm).

The concentration of intracellular calcium ions in the cells was calculated by use of the following formula.

$$Ca^{2+}\ (nM) = (\Delta F - F_{min}/F_{max} - \Delta F)Kd$$

$F_{min}$: Fluorescence intensity of Fluo3-AM when EDTA is added and $Ca^{2+}$ is blocked (minimum fluorescence intensity)

$F_{max}$: Fluorescence intensity of Fluo3-AM in the presence of excess $Ca^{2+}$ (breakage of cell membranes by a surfactant) (maximum fluorescence intensity)

Kd: Constant of dissociation of $Ca^{2+}$ from Fluo3-AM (390 nM)

(2) Detection of change in the concentration of intracellular calcium ions in cells, which occurs by means of capsaicin and ATP

Change in the concentration of intracellular calcium ions in epidermal keratinocytes was evaluated in the following cases: the case where epidermal keratinocytes were brought into contact with capsaicin, which is an agonist of a VR1 receptor; the case where epidermal keratinocytes were brought into contact with ATP, which is an agonist of a P2X receptor; the case where epidermal keratinocytes were brought into contact with αβ-methelene ATP (hereinafter referred to as "αβ-me ATP"), which is one of ATP analogues; and the case where epidermal keratinocytes were preliminarily treated with 2',3'-o-(2,4,6-trinitrophenyl)adenosine 5'-triphosphate (hereinafter referred to as "TNP-ATP"), which is one of ATP analogues and is an inhibitor of a P2X1 receptor, a P2X3 receptor, and a P2X2/3 receptor (each of the receptors is a P2X receptor subtype), and then the keratinocytes were brought into contact with αβ-me ATP.

Specifically, there were prepared a BSS containing capsaicin (final concentration: 100 nM to 10 μM) (hereinafter the solution may be referred to as a "capsaicin solution"), a BSS containing ATP (final concentration: 10 μM) (hereinafter the solution may be referred to as an "ATP solution"), and a BSS containing αβ-me ATP (final concentration: 10 μM) (hereinafter the solution may be referred to as an "αβ-me ATP solution"); and each of the solutions was added to epidermal keratinocytes. Separately, epidermal keratinocytes were preliminarily treated with TNP-ATP (100 nM) for 10 minutes, and the αβ-me ATP solution was added to the keratinocytes. After addition of each of the drug-containing BSSs to the epidermal keratinocytes, change in the concentration of intracellular calcium ions in the keratinocytes was measured over time by use of fluorescence intensity as an indication. The results are shown in Figs. 1A and 1B (in Figs. 1A and 1B, the vertical axis corresponds to the intracellular calcium ion concentration, and the horizontal axis corresponds to the concentration and type of drugs).

As shown in Fig. 1A, when the solution of capsaicin, which is an agonist of a VR1 receptor, is added to epidermal keratinocytes, the concentration of intracellular calcium ions in the keratinocytes is significantly increased. In contrast, as shown in Fig. 1B, when epidermal keratinocytes are preliminarily treated with capsazepine, which is an antagonist of a VR1 receptor, for 10 minutes, and then a solution of a mixture of capsaicin and capsazepine is added to the keratinocytes, an increase in the concentration of intracellular calcium ions in the keratinocytes is suppressed.

The results reveal that intracellular calcium ions enter epidermal keratinocytes by the mediation of a VR1 receptor.

As shown in Fig. 2, when ATP or αβ-me ATP, which is an agonist of a P2X receptor, is added to epidermal keratinocytes, the concentration of intracellular calcium ions in the keratinocytes is significantly increased. In contrast, when epidermal keratinocytes are preliminarily treated with TNP-ATP, which is an inhibitor of a P2X1 receptor, a P2X3 receptor, and a P2X2/3 receptor, for 10 minutes, and then a solution of a mixture of TNP-ATP and αβ-me ATP is added to the keratinocytes, an increase in the concentration of intracellular calcium ions in the keratinocytes is suppressed.

The results reveal that intracellular calcium ions enter epidermal keratinocytes by the mediation of a P2X receptor.

The above-described results reveal that the concentration of intracellular calcium ions in epidermal keratinocytes is increased through action of an agonist of a VR1 receptor or a P2X receptor in the keratinocytes, and such a phenomenon can be detected by means of the present detection method.

(3) Evaluation by use of various pain-producing substances

Utility of the present detection method and the present screening method was evaluated by use of various pain-producing substances.

(a) Bradykinin

Bradykinin, which is a typical pain-producing substance, was added to a BSS to thereby prepare bradykinin solutions of different bradykinin concentrations, each of the resultant solutions was added to epidermal keratinocytes, and, two seconds after addition of the solution, the concentration of intracellular calcium ions in the keratinocytes was calculated by use of the aforementioned formula.

The results are shown in Fig. 3 [wherein the vertical axis corresponds to the intracellular calcium ion concentration, and the horizontal axis corresponds to the bradykinin concentration (log M)]. As is clear from Fig. 3, when bradykinin is present ($10^{-8}$ M or more), the concentration of intracellular calcium ions in the epidermal keratinocytes is increased.

(b) Lactic acid

In a manner similar to that described above in (a), lactic acid was added to a BSS to thereby prepare lactic acid solutions of different lactic acid concentrations, each of the resultant solutions was added to epidermal keratinocytes, and, two seconds after addition of the solution, the concentration of intracellular calcium ions in the keratinocytes was calculated by use of the aforementioned formula.

The results are shown in Fig. 4 [wherein the vertical axis corresponds to the intracellular calcium ion concentration, and the horizontal axis corresponds to the lactic acid concentration (mM)]. As is clear from Fig. 4, when lactic acid is present, the concentration of intracellular calcium ions in the epidermal keratinocytes is increased.

As described above, the skin stimulation effect of a test substance can be detected by means of the present detection method employing, as an indication, an increase/decrease in the concentration of intracellular calcium ions in skin cells, and the present screening method, which utilizes the principle of the present detection method, is useful as means for screening, for example, various analgesic agents, anti-inflammatory agents, and drugs for preventing epidermal disorder.

Industrial Applicability

The present invention provides an excellent method of detecting the skin stimulation effect of a test substance; means for screening, for example, various analgesic agents, anti-inflammatory agents, and drugs for preventing epidermal disorder, the means employing the detection method; and means for evaluating conditions of the skin.

## Claims

**1.** A skin stimulation effect detection method comprising correlating the skin stimulation effect of a test substance which has been brought into contact with epithelial cells with an increase/decrease in the concentration of intracellular calcium ions in the epithelial cells, to thereby detect the skin stimulation effect of the test substance.

**2.** A skin stimulation effect detection method according to claim 1, wherein the epithelial cells are epidermal keratinocytes.

**3.** A skin stimulation effect detection method according to claim 1, wherein the test substance is a substance capable of changing the concentration of intracellular calcium ions in the epithelial cells by the mediation of a VR1 receptor and/or a P2X receptor on the epidermis.

**4.** A skin stimulation effect detection method according to claim 2, wherein the test substance is a substance capable of changing the concentration of intracellular calcium ions in the epithelial cells by the mediation of a VR1 receptor and/or a P2X receptor on the epidermis.

**5.** A method of screening drugs that are potentially useful for preventing skin stimulation and/or drugs that are potentially useful for preventing skin disorder, which comprises using a skin stimulation effect detection method as recited in any of claims 1 through 4 as screening means and, as an indication, suppression of an increase in the concentration of intracellular calcium ions in epithelial cells, which increase is caused by a skin stimulation substance.

**6.** A method of evaluating conditions of the skin, which comprises correlating an increase/decrease in the concentration of intracellular calcium ions in epidermal cells with conditions of the skin.

Fig. 1A

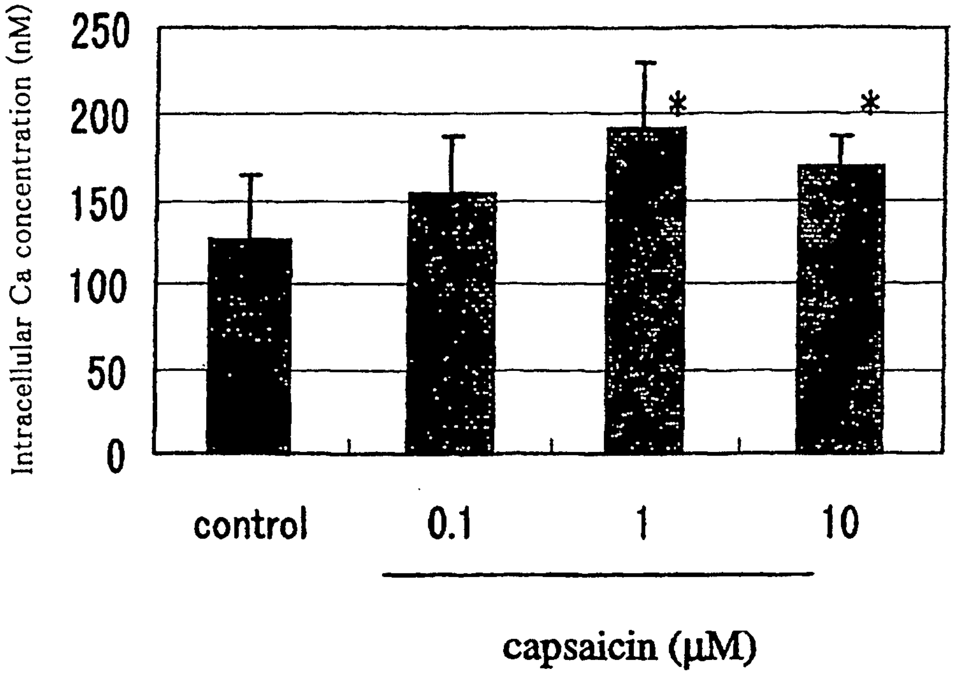
Intracellular Ca concentration (nM)
250
200
150
100
50
0
*
*
control
0.1
1
10
capsaicin (μM)

Fig. 1B

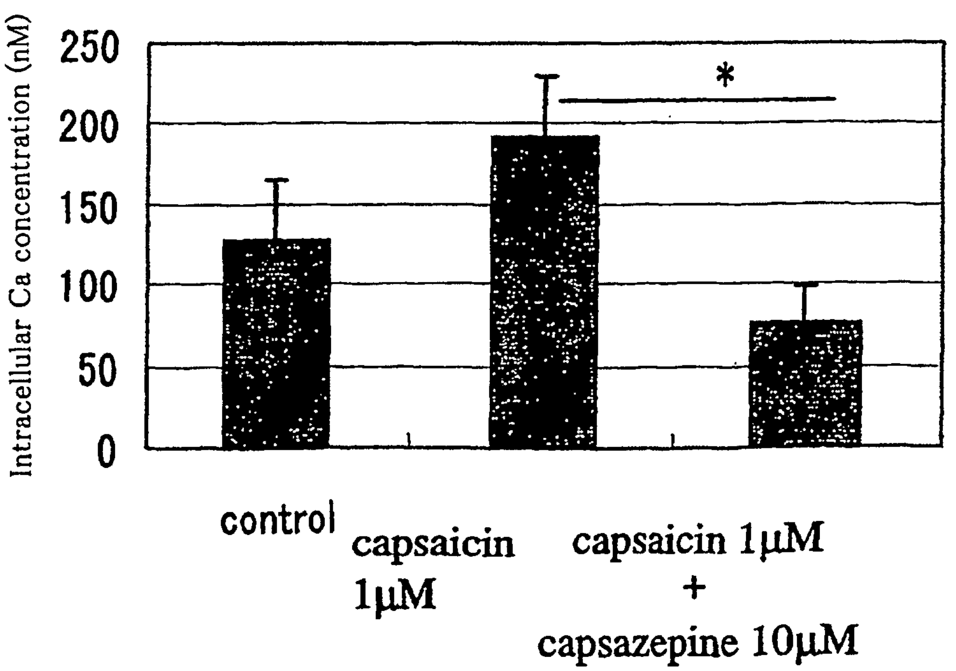
Intracellular Ca concentration (nM)
250
200
150
100
50
0
*
control
capsaicin 1μM
capsaicin 1μM + capsazepine 10μM

Fig. 2

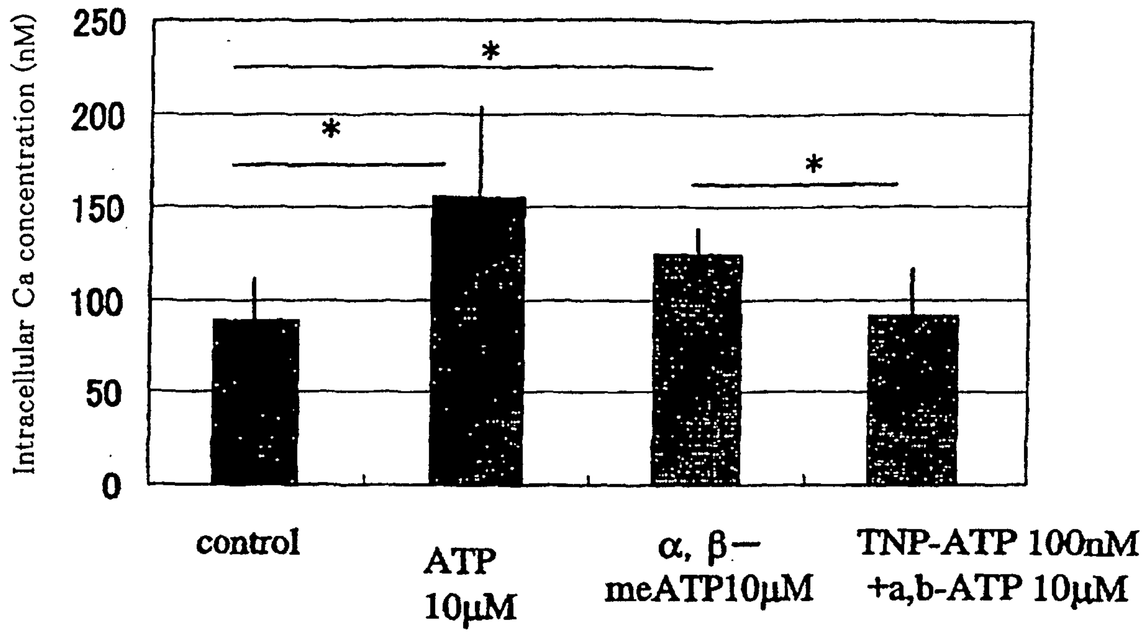
Intracellular Ca concentration (nM)
250
200
150
100
50
0
*
*
*
control
ATP 10μM
α, β−meATP10μM
TNP-ATP 100nM +a,b-ATP 10μM

Fig. 3

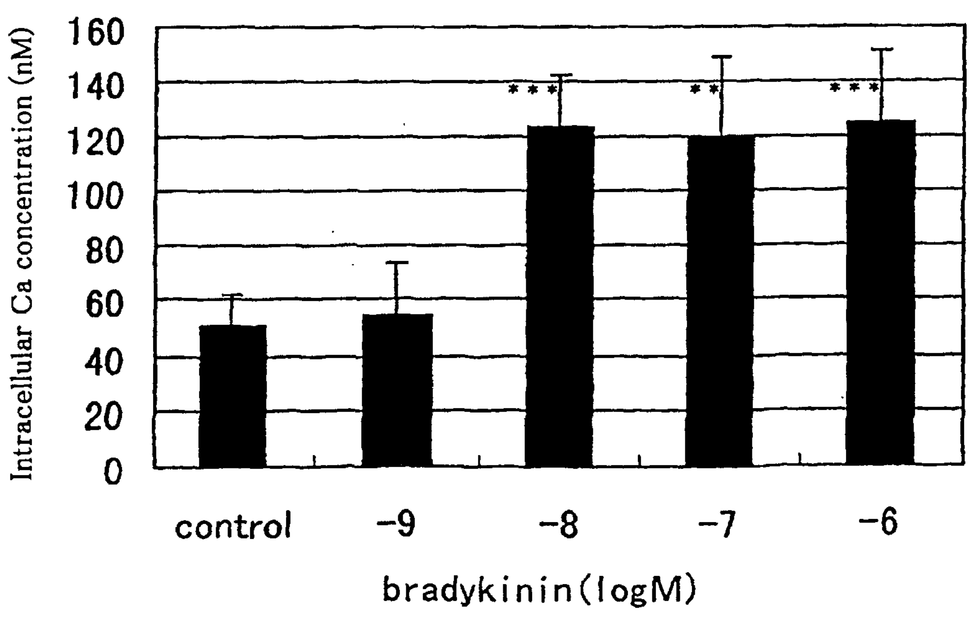
Intracellular Ca concentration (nM)
160
140
120
100
80
60
40
20
0
***
**
***
control
−9
−8
−7
−6
bradykinin(logM)

Fig. 4

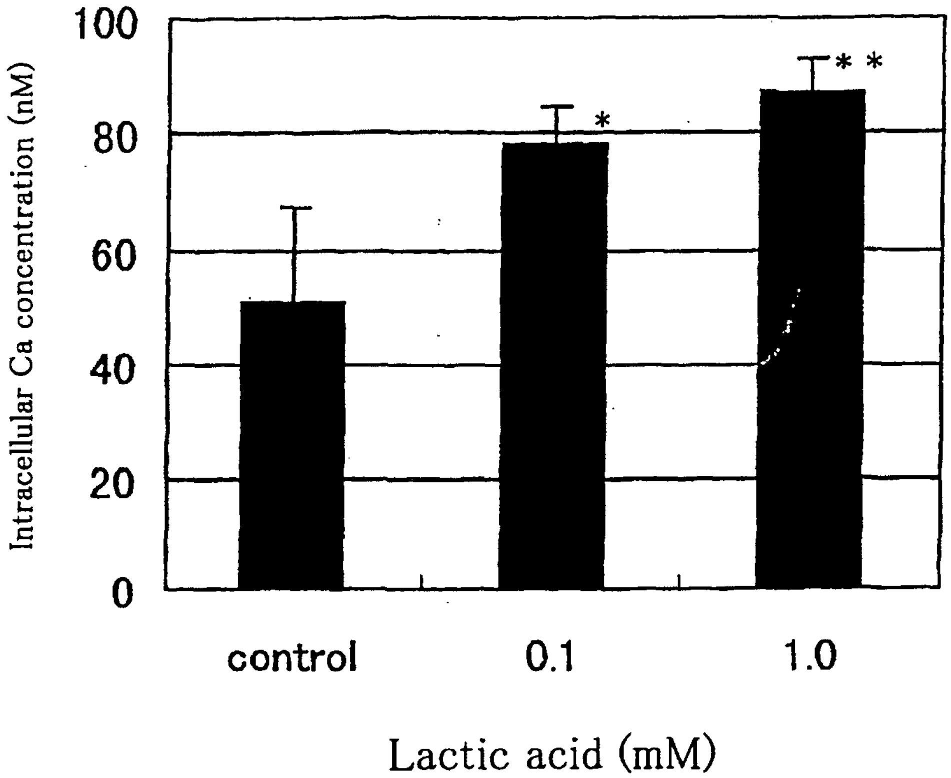
100
80
60
40
20
0
Intracellular Ca concentration (nM)
*
* *
control
0.1
1.0
Lactic acid (mM)

| INTERNATIONAL SEARCH REPORT | International application No. PCT/JP02/05736 |
|---|---|

A. CLASSIFICATION OF SUBJECT MATTER
Int.Cl[7] G01N33/15, G01N33/50, A61P17/00, A61K49/00, A61K45/00

According to International Patent Classification (IPC) or to both national classification and IPC

B. FIELDS SEARCHED

Minimum documentation searched (classification system followed by classification symbols)
Int.Cl[7] G01N33/15, G01N33/50, A61P17/00, A61K49/00, A61K45/00

Documentation searched other than minimum documentation to the extent that such documents are included in the fields searched

| | | | |
|---|---|---|---|
| Jitsuyo Shinan Koho | 1922-1996 | Toroku Jitsuyo Shinan Koho | 1994-2002 |
| Kokai Jitsuyo Shinan Koho | 1971-2002 | Jitsuyo Shinan Toroku Koho | 1996-2002 |

Electronic data base consulted during the international search (name of data base and, where practicable, search terms used)

C. DOCUMENTS CONSIDERED TO BE RELEVANT

| Category* | Citation of document, with indication, where appropriate, of the relevant passages | Relevant to claim No. |
|---|---|---|
| X | JP 2000-297015 A (Taisho Pharmaceutical Co., Ltd.), 24 October, 2000 (24.10.00), & WO 00/47172 A1 & AU 2459100 A | 1-6 |
| A | JP 11-49647 A (Shiseido Co., Ltd.), 23 February, 1999 (23.02.99), (Family: none) | 1-6 |
| A | JP 10-229978 A (Shiseido Co., Ltd.), 02 September, 1998 (02.09.98), (Family: none) | 1-6 |
| P,A | JP 2002-62294 A (Shiseido Co., Ltd.), 28 February, 2002 (28.02.02), (Family: none) | 1-6 |

☐ Further documents are listed in the continuation of Box C. ☐ See patent family annex.

* Special categories of cited documents:
"A" document defining the general state of the art which is not considered to be of particular relevance
"E" earlier document but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 02 September, 2002 (02.09.02) | 17 September, 2002 (17.09.02) |
| Name and mailing address of the ISA/ Japanese Patent Office | Authorized officer |
| Facsimile No. | Telephone No. |

Form PCT/ISA/210 (second sheet) (July 1998)